## Europäisches Patentamt
## European Patent Office
## Office européen des brevets

(19)

(11) Veröffentlichungsnummer: **0 045 718**
**A2**

(12)

# EUROPÄISCHE PATENTANMELDUNG

(21) Anmeldenummer: **81810295.6**

(51) Int. Cl.³: **A 61 K 9/06**

(22) Anmeldetag: **20.07.81**

(30) Priorität: **25.07.80 CH 5714/80**

(43) Veröffentlichungstag der Anmeldung:
**10.02.82 Patentblatt 82/6**

(84) Benannte Vertragsstaaten:
**AT BE CH DE FR GB IT LI LU NL SE**

(71) Anmelder: **CIBA-GEIGY AG**
**Patentabteilung Postfach**
**CH-4002 Basel(CH)**

(72) Erfinder: **Schmidt-Ruppin, Karl Heinz, Dr.**
**Im Baumgarten 5**
**CH-4144 Arlesheim(CH)**

(54) **Nasalpräparate und Verfahren zu deren Herstellung.**

(57) Die vorliegende Erfindung betrifft neue Nasalpräparate zur Prophylaxe gegen Infektionen durch Viren des Respirationstraktes, vor allem durch Influenza-A-Viren. Diese Präparate enthalten als Wirkstoffe ein oder mehrere Glucane, die mindestens teilweise aus aufeinanderfolgenden $(1\rightarrow3)$-$\beta$-D-Glucose-Einheiten bestehen und gegebenenfalls partiell abgebaut und/oder partiell durch Niederalkyl, Hydroxyniederalkyl oder Carboxyniederalkyl veräthert sind. Ebenfalls Gegenstand der Erfindung ist die Herstellung sowie die Verwendung dieser Präparate.

EP 0 045 718 A2

- 1 -

CIBA-GEIGY AG                    4-12984/+

Basel (Schweiz)

## Nasalpräparate und Verfahren zu deren Herstellung

Die vorliegende Erfindung betrifft neue Nasalpräparate zur Prophylaxe gegen Infektionen durch Viren des Respirationstraktes, vor allem durch Influenza-A-Viren, Verfahren zur Herstellung dieser Präparate sowie die Verwendung dieser Präparate.

Es wurde überraschenderweise gefunden, dass Glucane, die mindestens teilweise aus aufeinanderfolgenden $(1 \rightarrow 3)$-$\beta$-D-Glucose-Einheiten bestehen und gegebenenfalls partiell abgebaut und/oder partiell durch Niederalkyl, Hydroxyniederalkyl oder Carboxyniederalkyl veräthert sind, wie z.B. die weiter unten näher beschriebenen, definitionsgemässen Glucane aus Bakterien, Pilzen oder Hefen, bei intranasaler Applikation eine mehrere Tage lang dauernde prophylaktische Wirkung gegenüber Infektionen durch Viren des Respirationstraktes, insbesondere Influenza-A-Viren, ausüben.

Gegenstand der vorliegenden Erfindung sind deshalb Nasalpräparate zur Prophylaxe gegen Infektionen durch Viren des Respirationstraktes, die als Wirkstoff ein oder mehrere der vorstehend definierten Glucane enthalten. Die erfindungsgemässen Präparate sind insbesondere übliche Arzneiformen für die intranasale Anwendung.

Als Wirkstoffe kommen beispielsweise als definitionsgemässes Glucan aus Pilzen das nicht-tumorhemmende Pachyman aus Poria cocos Wolf (Bukuryo), vgl. G. Chihara et al., Nature 225, 943-944 (1970), welches neben $(1 \rightarrow 3)$-$\beta$-glykosidischen Bindungen noch $(1 \rightarrow 6)$-$\beta$-glykosidische Bindungen aufweist, weiter beispielsweise definitionsgemässe Derivate davon wie Hydroxyäthylpachyman (vgl. J. Hamuro et al.,

Nature 245, 40 (1973), Hydroxypropylpachyman, sowie das aus Pachyman durch aufeinanderfolgende Oxidation mit Natriumperoxid, Reduktion mit Natriumborhydrid und milde Hydrolyse mit 0,05-mol. Schwefelsäure bei Raumtemperatur unter Beseitigung der (1 → 6)-β-glykosidischen Bindungen entstehende tumorhemmende Pachymaran, vgl. G. Chihara et al., s. oben, ferner das davon abgeleitete Carboxymethylpachymaran, vgl. Hamuro et al., Nature 233, 486 (1971), und als definitionsgemässe Glucane aus Bakterien z.B. das tumorhemmende (1 → 3)-β-D-Glucan aus Alcaligenes faecalis var. myxogenes, IFO 13140, und seine durch Säureeinwirkung erhaltenen Abbauprodukte von verschiedener Kettenlänge, vgl. T. Harada et al., Arch. Biochem. Biophys. 124, 292-298 (1968), J. Nakanishi, Carbohydrate Res. 32, 47-52 (1974), H. Saito et al., Agr. Biol. Chem. (Tokio), 32, 1261-1269 (1968) und bezüglich tumorhemmender Wirkung besonders T. Sasake et al., Cancer Research 38, 379-383 (1968) , in Betracht.

Als bei intranasaler Applikation gegen Infektionen durch Influenza-A-Viren prophylaktisch wirkende definitionsgemässe Glucane aus Hefen seien entsprechende Glucane und Glucangemische aus Zellwänden von Saccharomyces cerevisiae genannt, vgl. hierzu R.L. Whistler, A.A. Bushway, P.R. Singh, W. Nakahara und R. Tokuzen, Adv. Carbohydrate Biochem. 32, 235 (1976) sowie, besonders bezüglich deren tumorhemmender Wirkung, N.R. Di Luzio, J. A. Cook, C. Cohen, J. Rodrigue und E. Jones in "Macrophage Cancer", Editoren K. James, B. McBride und A. Stewart, Univ. of Edinburgh, Medical School Press, Edinburgh 1977, page 188, und, besonders bezüglich der antifungalen Wirkung gegen Candida albicans, D.L. Williams, J.A. Cook, E.O. Hoffman und N.R. Di Luzio, J. Rethiculoendothel. Soc. 23, 479 (1978).

Die prophylaktische Wirkung von definitionsgemässen Glucanen gegen Infektionen durch Influenza A-Viren geht beispielsweise aus den nachstehend beschriebenen Versuchen hervor.

## Methode

Männliche NMRI-Mäuse von 16 bis 18 g Körpergewicht, eingeteilt in Gruppen von je 10, werden mit einer für 90% der Tiere tödlichen Suspension von Influenza A/Hongkong 1/68-Virus ($H_3N_2$) in 0,05 ml Hank's Lösung unter leichter Aethernarkose intranasal infiziert. Zuvor werden die Mäuse der verschiedenen Gruppen, ausgenommen der zwei Kontrollgruppen, durch intranasale Applikation von je 0,05 ml Lösung des zu prüfenden Wirkstoffes in verschiedenen Konzentrationen in destilliertem Wasser zu verschiedenen, in der Tabelle I angegebenen Zeiten ein- oder mehrmals vorbehandelt. Die Wirkung der Vorbehandlungen wird anhand der Prozentzahl der nach 15 Tagen überlebenden Mäuse im Vergleich zu den Kontrollgruppen, und anhand der Verlängerung der mittleren Ueberlebenszeit innerhalb einer Beobachtungsperiode von 15 Tagen im Vergleich zu den Kontrollgruppen festgestellt. Die Resultate sind in der nachstehenden Tabelle I zusammengestellt.

| Wirkstoff-Konzentration | Applikationszeit Tage (D) bzw. Minuten (M) vor Infektion | | | Schutzwirkung gegen Influenza-Virus A Hongkong 1/68 Ueberlebende in %,Test/Kontrollen | Mittlere Ueberlebenszeit in Tagen,Test/Kontrollen |
|---|---|---|---|---|---|
| Pachyman | -D7 | | | 90/10** | 14.1/9,6*** |
| 1 % | | -D3 | | 90/10** | 14,6/9,6*** |
| | | | -M30 | 70/10* | 12,2/9,6* |
| | -D7 | -D3 | | 70/10* | 13,0/9,6* |
| | | -D3 | -M30 | 70/10* | 12,5/9,6* |
| 0,4% | -D7 | | | 80/10** | 13,6/9,6** |
| | | -D3 | | 60/10 | 11,9/9,6 |
| | | | -M30 | 70/10* | 13,1/9,6* |
| | -D7 | -D3 | | 90/10** | 14,2/9,6*** |
| | | -D3 | -M30 | 100/10** | >15,0/9,6*** |
| 0,1% | -D7 | | | 60/10 | 11,8/9,6 |
| | | -D3 | | 70/10* | 13,1/9,6* |
| | | | -M30 | 80/10** | 13,8/9,6** |
| | -D7 | -D3 | | 40/10 | 10,6/9,6 |
| | | -D3 | -M30 | 80/10** | 14,4/9,6*** |
| (1—3)-β-D-Glucan[1]) | -D7 | | | 60/10 | 11,8/9,6 |
| 1% | | -D3 | | 40/10 | 10,3/9,6 |
| | | | -M30 | 50/10 | 11,4/9,6 |
| | -D7 | -D3 | | 66/10 | 12,2/9,6* |
| | | -D3 | -M30 | 66/10 | 12,2/9,6* |

- 5 -

| * | Signifikant $P < 0,05$ | Contingency-Test für Ueberlebende |
| ** | Signifikant $P < 0,01$ | bzw. Cox-Test für mittlere |
| *** | Signifikant $P < 0,001$ | Ueberlebenszeit |

1) Aus Alcaligenes faecalis, var. myxogenes, IFO 13140.

Aus den in der Tabelle I zusammengestellten Resultaten geht hervor, dass Pachyman in den Konzentrationen von 1 % und 0,4 % schon bei einmaliger Applikation, wenn diese 3 oder 7 Tage vor der Infektion liegt, eine starke Schutzwirkung gegen Influenza A-Viren gewährleistet. Nur wenig schwächer ist die Schutzwirkung bei einmaliger Applikation von Pachyman in denselben Konzentrationen 30 Minuten vor der Infektion, sowie generell bei Verabreichung von Pachyman in der Konzentration von 0,1 %, und bei Verabreichung des $(1 \longrightarrow 3)$-$\beta$-Glucans aus Alcaligenes faecalis, var. myxogenes IFO 13140 in der Konzentration von 1 %.

Gleichartige Schutzeffekte wie die vorstehenden gegen Influenzavirus A/Hongkong 1/68 ($H_3N_2$) fanden sich auch gegen Infektionen mit Influenzavirus A/Vict. 3/75 ($H_3N_2$) und auch bei weiteren definitionsgemässen Glucanen, wie z.B. solchen aus Saccharomyces cerevisiae.

Diese Wirkungen der geprüften Glucane, insbesondere von Pachyman, bei nasaler Verabreichung übertreffen die prophylaktische Wirkung von bekannten antiviral wirksamen Stoffen, wie Amantadin, gegenüber Influenza A-Viren.

Die erfindungsgemässen Nasalpräparate sind insbesondere mit üblichen Hilfsstoffen bereitete Nasaltropfen, Nasalsprays, Nasalgele und Nasalsalben. Diese enthalten den Wirkstoff in einer prophylaktischen wirksamen, d.h. eine Schutzwirkung gegen Virusinfektionen der oben besagten Art gewährleistenden Menge und Konzentration. Entsprechend enthalten sie den Wirkstoff in einer für eine Verabreichung von mindestens 2 mg Wirkstoff pro Applikation geeigneten Konzentration. Die obere Grenze der letzteren liegt bei 4 % (G/V) und die untere Grenze, - weil eine einzelne Verabreichung nicht nur durch einmalige nasale Applikation, sondern auch durch nach Antrocknen ein- oder mehrmals wiederholte Applikation erfolgen kann - bei etwa 0,2% (G/V), entsprechend 1 ml eines gebrauchsfertigen Nasalpräparates für die obige Mindestdosis. Nasaltropfen enthalten insbesondere zwischen 1 und 2%, und Nasalsprays insbesondere 0,2 bis 1% Wirkstoff. In beiden Fällen dient als Lösungsmittel vorzugsweise Wasser oder eine physiologische Lösung; die wässrigen Lösungen enthalten gegebenenfalls übliche pharmazeutisch annehmbare Zusätze zur Stabilisierung des Wirkstoffes, Pufferung, Konservierung und/oder Herabsetzung der Oberflächenspannung und können in üblicher Weise, z.B. mit Natriumchlorid oder Pufferlösungen, isotonisch gemacht sein.

Die Erfindung betrifft in erster Linie mit obengenannten Lösungen gefüllte Sprayfläschchen und ähnliche zur intranasalen Applikation solcher Lösungen geeignete Behälter.

Die Anwendung der erfindungsgemässen Mittel zur Prophylaxe gegen Influenza A und Infektionen durch andere Viren des Respirationstraktes erfolgt vorzugsweise in Abständen von 2 bis 3 Tagen bzw. zweimal wöchentlich. Statt für eine länger, z.B. die ganze kalte Jahreszeit dauernde Prophylaxe können die erfindungsgemässen Mittel auch nur während eines Teiles der kalten Jahreszeit bei offensichtlich erhöhter Ansteckungsgefahr angewendet werden. Weiter können sie auch zur Ueberbrückung der Latenzphase bei Influenza-Schutzimpfungen, d.h. gleichzeitig mit oder unmittelbar vor der Impfung, angewendet werden, da sie bei gleichzeitiger nasaler Applikation mit parenteral injizierter Vakzine einen Schutz in der Antikörper-freien Latenzphase gewähren, ohne die Antikörperbildung zu beeinträchtigen. Dadurch kann das Risiko einer Infektion innerhalb der ersten beiden Wochen nach der Vakzinierung vermindert werden.

Nachfolgend folgen Beispiele für Applikationsformen. Diese Beispiele sollen jedoch den Umfang der Erfindung in keiner Weise
beschränken.


Beispiel 1: Nasalspray

Zur Bereitung eines Nasalsprays mit 1% Wirkstoffgehalt löst
man 10,0 g Pachyman in 1 Liter destilliertem Wasser und füllt die
Lösung, gewünschtenfalls nach Zusatz von 10 mg Natrium-timerfonat
[Natrium-p-(äthylmercurithio)-benzolsulfonat), in Spray-Fläschchen
von z.B. 10 ml Inhalt. Zur Prophylaxe gegen Influenza und andere
Infektionen durch Viren des Respirationstraktes werden z.B. jeden 2.
oder 3. Tag oder zweimal wöchentlich aus vollem Fläschchen 2-3 Druckstösse, aus teilweise entleertem Fläschchen nötigenfalls mehr, in
jede Nasenhälfte gespritzt.


Beispiel 2: Nasaltropfen

Zur Bereitung von Nasaltropfen mit 1% Wirkstoffgehalt löst
man 10,0 g Pachyman in 1 Liter destilliertem Wasser und füllt die
Lösung, gewünschtenfalls nach Zusatz von 10 mg Natrium-timerfonat, in
mit einem als Pipette ausgestalteten Stopfen versehene Tropffläschchen
von z.B. 5 oder 10 ml Inhalt. In gleichen Intervallen, wie im Beispiel 1 angegeben, appliziert man je 4-6 Tropfen, entsprechend ca.
0,2 oder 0,3 ml, enthaltende ca. 2-3 mg Wirkstoff, in jede Nasenhälfte.


Anstelle von Pachyman kann man in diesem Beispiel und ebenso
im Beispiel 1 auch die gleiche Menge von $(1 \rightarrow 3)$-β-D-Glucan aus
Alcaligenes faecalis var. myxogenes IFO 13140 verwenden.

Patentansprüche

1.     Nasalpräparate zur Prophylaxe gegen Infektionen durch Viren
des Respirationstraktes, dadurch gekennzeichnet, dass sie als Wirkstoff mindestens ein Glucan, das mindestens teilweise aus aufeinanderfolgenden (1 → 3)-β-D-Glucose-Einheiten besteht und gegebenenfalls
partiell abgebaut und/oder partiell durch Niederalkyl, Hydroxyniederalkyl oder Carboxyniederalkyl veräthert ist, enthalten.

2.     Nasalpräparate gemäss Anspruch 1 in Form von üblichen Arzneiformen für die intranasale Anwendung.

3.     Nasalpräparate gemäss Anspruch 1 in Form von Nasalsprays.

4.     Nasalpräparate gemäss Anspruch 1 in Form von Nasaltropfen.

5.     Nasalpräparate gemäss Anspruch 1 in Form von Nasalgelen.

6.     Nasalpräparate gemäss Anspruch 1 in Form von Nasalsalben.

7.     Nasalpräparate gemäss Anspruch 1, bestehend aus einem mit
einer wässrigen Lösung eines Wirkstoffes gemäss Anspruch 1 und gegebenenfalls von üblichen Zusatzstoffen in einem zur nasalen Applikation einer Flüssigkeit geeigneten Behälter.

8.     Nasalpräparate gemäss einem der Ansprüche 1 bis 7, gekennzeichnet durch einen Gehalt an dem Glucan Pachyman.

9.     Nasalpräparate gemäss einem der Ansprüche 1 bis 7, gekennzeichnet durch einen Gehalt an dem (1 →3)-β-D-Glucan aus Alcaligenes
faecalis var. myxogenes IFO 13140.

10.     Nasalpräparate gemäss einem der Ansprüche 1 bis 7, gekennzeichnet durch einen Gehalt von 0,2 bis 4% Pachyman.

11.     Nasalpräparate gemäss einem der Ansprüche 1 bis 7, gekennzeichnet durch einen Gehalt von 0,2 bis 4% (1 ⟶ 3)-β-D-Glucan aus Alcaligenes faecalis var. myxogenes IFO 13140.

12.     Nasalpräparate gemäss Ansprüchen 2, 7 und 8, gekennzeichnet durch einen Gehalt an etwa 0,2 bis etwa 1% Pachyman.

13.     Nasalpräparate gemäss Ansprüchen 2, 7 und 9, gekennzeichnet durch einen Gehalt an etwa 0,2 bis etwa 1% (1 ⟶ 3)-β-D-Glucan aus Alcaligenes faecalis var. myxogenes IFO 13140.

14.     Nasalpräparate gemäss Ansprüchen 4 und 8, gekennzeichnet durch einen Gehalt an etwa 1 bis etwa 2% Pachyman.

15.     Nasalpräparate gemäss Ansprüchen 4 und 9, gekennzeichnet durch einen Gehalt an etwa 1 bis etwa 2% (1 ⟶ 3)-β-D-Glucan aus Alcaligenes faecalis var. myxogenes IFO 13140.

16.     Verfahren zur Herstellung von Nasalpräparaten zur Prophylaxe gegen Infektionen durch Viren des Respirationstraktes, dadurch gekennzeichnet, dass man mindestens ein Glucan, das mindestens teilweise aus aufeinanderfolgenden (1 ⟶ 3)-β-D-glucose-Einheiten besteht und gegebenenfalls partiell abgebaut und/oder partiell durch Niederalkyl, Hydroxyniederalkyl oder Carboxyniederalkyl veräthert ist, in Wasser löst, gegebenenfalls übliche Zusatzstoffe beifügt und gewünschtenfalls die erhaltene Lösung in zur nasalen Applikation einer Flüssigkeit geeignete Behälter abfüllt.

17.     Verfahren nach Anspruch 16, dadurch gekennzeichnet, dass man als Glucan Pachyman in einer eine Konzentration von etwa 0,5 bis etwa 2% ergebenden Menge verwendet.

18.   Verfahren nach Anspruch 16, dadurch gekennzeichnet, dass man als Glucan das $(1 \rightarrow 3)$-$\beta$-D-Glucan aus Alcaligenes faecalis var. myxogenes IFO 13140 in einer Konzentration von etwa 0,5 bis etwa 2% ergebenden Menge verwendet.

19.   Verfahren nach Anspruch 16, dadurch gekennzeichnet, dass man als Glucan Pachyman   in einer eine Konzentration von etwa 1% ergebenden Menge verwendet.

20.   Verfahren nach Anspruch 16, dadurch gekennzeichnet, dass man als Glucan das $(1 \rightarrow 3)$-$\beta$-D-Glucan aus Alcaligenes faecalis myxogenes IFO 13140 in einer eine Konzentration von etwa 1% ergebenden Menge verwendet.

21.   Verwendung von Nasalpräparaten gemäss einem der Ansprüche 1 bis 15 zur Prophylaxe gegen Infektionen durch Viren des Respirationstraktes.

22.   Verwendung von Nasalpräparaten gemäss einem der Ansprüche 1 bis 15 zur Ueberbrückung der Latenzphase bei Influenza-Schutzimpfungen.